Europäisches Patentamt

European Patent Office

Office européen des brevets

(19)

(11) Veröffentlichungsnummer: **0 002 775**
**B1**

(12) **EUROPÄISCHE PATENTSCHRIFT**

(45) Veröffentlichungstag der Patentschrift:
21.04.82

(51) Int. Cl.³: **A 61 M 5/14**, G 06 F 15/42

(21) Anmeldenummer: 78101750.4

(22) Anmeldetag: 18.12.78

(54) Vorrichtung zur vorprogrammierbaren Infusion von Flüssigkeiten.

(30) Priorität: 28.12.77 DE 2758368

(43) Veröffentlichungstag der Anmeldung:
11.07.79 Patentblatt 79/14

(45) Bekanntmachung des Hinweises auf die Patenterteilung:
21.04.82 Patentblatt 82/16

(84) Benannte Vertragsstaaten:
CH DE FR GB SE

(56) Entgegenhaltungen:
DE-A-2 451 424
DE-A-2 513 467
US-A-3 809 871

(73) Patentinhaber: SIEMENS AKTIENGESELLSCHAFT Berlin und München, Postfach 22 02 61, D-8000 München 22 (DE)

(72) Erfinder: Franetzki, Manfred, Dr., Schleifweg 7b, D-8521 Uttenreuth (DE)
Erfinder: Gagneur, Klaus, Wiesenweg 11, D-8521 Bubenreuth (DE)
Erfinder: Prestele, Karl, Leipziger Strasse 74, D-8520 Erlangen (DE)

## Vorrichtung zur vorprogrammierbaren Infusion von Flüssigkeiten

Die Erfindung bezieht sich auf eine Vorrichtung zur vorprogrammierbaren Infusion von Flüssigkeiten in einen Patientenkörper, insbesondere zur Insulinverabreichung bei der Diabetes-Therapie, bestehend aus einer Mikrodosiereinheit für die Flüssigkeit sowie einer der Mikrodosiereinheit vorgeschalteten, programmierbaren und mit einem Programmspeicher für die Mikrodosiereinheit versehenen Steuervorrichtung, in die ein Steuerprogramm für den Verlauf der Infusionsrate zwischen vorgegebenen Zeitpunkten eines 24-Stunden-Rhythmus manuell einprogrammierbar ist.

Bei der Diabetes-Therapie ist es erwünscht, Insulin fortwährend in unterschiedlichen Raten in den Körper des Patienten zu infundieren, weil der Insulinbedarf des Zuckerkranken während des Tages, bedingt z. B. durch den Rhythmus der Mahlzeiten, großen Schwankungen unterworfen ist. Es hat sich gezeigt, daß — solange noch keine mittels Glucosesensor selbsttätig regelnden Infusionsgeräte zur Verfügung stehen — am günstigsten die Insulinabgabe nach einem für den Patienten individuell einstellbaren und vorprogrammierbaren Tagesprofil erfolgen sollte. Aus der DE-A-2 451 424 ist ein Gerät für die Zuführung von Flüssigkeiten zum menschlichen oder tierischen Körpern bekannt, bei dem ein elektrischer Programmgeber vorhanden ist, der von einer Bedienungsperson mit manuell einstellbaren Steuersignalen unterschiedlicher Größe auf ein sich über vorgegebene Zeitschritte erstreckendes Programm vorprogrammierbar ist. Die Steuersignale werden dabei zweckmäßigerweise mittels Kreuzschienenverteiler eingestellt. Das Gerät nach der DE-A-2 451 424 ist speziell als »Bedside-Gerät« konzipiert. Aus der DE-A-2 513 467 ist darüber hinaus auch bereits ein Infusionsgerät vorbekannt, das insbesondere in den Patientenkörper implantierbar sein soll, wobei im Sinne der Verabreichung eines gewünschten Tagesprofils der Infusion die Steuerung von einem gegebenenfalls extrakorporalen Steuergerät erfolgen kann. Dabei enthält das Steuergerät Speichermittel und auch einen speziellen Programmgeber. Eine Übertragung der Programmiereinrichtung der DE-A-2 451 424 für ein Gerät nach der DE-A-2 513 467 würde bedeuten, ein Programmiergerät tragbar zu gestalten. Dies würde für den Patienten eine unzumutbare Belastung sein und überdies die Möglichkeit einer unbefugten Manipulation am Programmiergerät beinhalten.

Andererseits muß in jedem Fall bei solchen programmierbaren, extrakorporal oder implantierbaren Infusionsgeräten mit Mikrodosiereinheit gewährleistet sein, daß eine Vorprogrammierung des erwünschten Tagesprofils vom Arzt in einfacher und übersichtlicher Weise erfolgen kann. Bei der Behandlung von Diabetikern treten z. B. Fälle auf, bei denen erst innerhalb einer längeren Untersuchungsperiode, während der sich der Patient unter ständiger ärztlicher Kontrolle befindet, ein optimales Tagesprofil des Insulinbedarfs für ein später zu implantierendes Infusionsgerät oder auch für eine spätere herkömmliche Injektionstherapie ermittelt werden soll. Während solcher Untersuchungsperioden soll sich der Patient Tag und Nacht während der Insulinzufuhr frei bewegen können. Für derartige Anwendungszwecke sind die vom Stand der Technik bekannten Geräte nicht optimal geeignet.

Aufgabe der Erfindung ist es daher, obigen Mangel zu beseitigen. Es soll eine weitere, verbesserte Vorrichtung zur Infusion von Flüssigkeiten in den menschlichen oder tierischen Körper geschaffen werden, die weitestgehend die angestrebten Ziele von tragbaren Infusionsgeräten mit selbsttätiger Regelung mit den spezifischen Vorteilen von unter unmittelbarer Aufsicht eines Arztes stehenden »Bedside-Geräten« mit vielfältigen Kontroll- und Eingriffsmöglichkeiten vereinigt. Dabei soll insbesondere die Mikrodosiereinheit mit zugehöriger Steuervorrichtung bequem am Patientenkörper tragbar sein, wobei dem Arzt die Möglichkeit gegeben sein muß, das in ausreichend kleinen Zeitrastern im 24-Stunden-Rhythmus mit diskreten Infusionsraten vorprogrammierte Steuerprogramm zu ändern und an den jeweiligen Bedarf anzupassen.

Die Aufgabe ist erfindungsgemäß dadurch gelöst, daß zur Programmierung ein von der Steuervorrichtung getrenntes, eigenes Programmiergerät vorhanden ist, und daß der Speicher für das vorprogrammierte Steuerprogramm der Steuervorrichtung als Programmgeber für die Mikrodosiereinheit vorgesehen und ausschließlich zur Übernahme des vorprogrammierten Steuerprogramms kurzzeitig mit dem externen Programmiergerät verbindbar ist.

Die nun geschaffene Infusionsvorrichtung besteht im wesentlichen aus in Funktionsgruppen getrennten Einzelgeräten: Der Mikrodosiereinheit, der zugehörigen Steuervorrichtung und dem externen Programmiergerät. Durch die Trennung in diese Funktionseinheiten wird der Einsatz der Infusionsvorrichtung variabler. Die Mikrodosiereinheit mit zugehöriger Steuereinheit werden vom Patienten am Körper getragen, während das Programmiergerät stationär für den Arzt für andere Aufgaben zur Verfügung steht. Dabei ist die Mikrodosiereinheit beispielsweise unmittelbar fest am Körper des Patienten zur Infusion angeordnet, während die Steuervorrichtung leicht zugänglich in einer Tasche od. dgl. getragen wird. Fakultativ kann die Mikrodosiereinheit im Körper des Patienten implantiert sein. Vom Arzt wird am stationären Programmiergerät bei Bedarf für einen Patienten ein den individuellen Bedürfnissen entsprechendes Tagesprofil einprogrammiert, das dann als Festprogramm durch kurzzeitige Verbindung vom Programmiergerät und Steuergerät in die internen Speichermittel der Steuervorrichtung übernommen wird und für längere Zeiträume die Steuerung der Mikrodosiereinheit übernimmt.

Vorzugsweise ist das Programmiergerät als elektrischer Verteiler, beispielsweise Kreuzschienenverteiler, mit manuell einstellbaren Steuerpunkten, ausgebildet. Alternativ dazu kann dieser Verteiler als Infusionsratenschalter ausgebildet sein, mit dem vom Arzt diskrete Infusionsraten zeitlich durchgetaktet entsprechend einem zeitlichen aufeinanderfolgenden Ablauf eingestellt werden können. In vorteilhafter Weiterbildung wird mit dem Programmiergerät ein Tagesprofil der Dosierung auf separate Programmträger gegeben, die von der Steuervorrichtung mittels Leseeinheit zur Übernahme in die internen Speicher lesbar sind. Als Programmträger kommen Lochkarten, Lochstreifen oder Magnetkarten in Frage. Insbesondere Lochstreifen sind als Informationsträger für die Speicherung von Dosierungsprofilen einer zeitlich vorgegebenen Flüssigkeitsinfusion aus der US-A-3 809 871 vorbekannt.

Eine derartige Weiterbildung hat den Vorteil, daß bei geeigneter Codierung das Tagesprofil der Infusion auf Lochkarten oder Lochstreifen gestanzt praktisch als Analogkurve darstellbar ist, die vom Benutzer sofort optisch erkannt und klassifiziert werden kann, wobei derartige Lochkarten oder Lochstreifen in einfacher Weise archiviert werden können. Es lassen sich so entsprechende Programmbibliotheken aufbauen, aus denen der Zugriff zu einem Einzelprogramm schnell erfolgen kann. Wird das Tagesprofil der Dosierung codiert auf Magnetkarten od. dgl. gegeben, ist es sonnvoll, die Analogkurve der Dosierung zur Kontrolle auf einen an das Programmiergerät anschließbaren Registrierer zu geben.

In beiden Fällen weist die Steuervorrichtung zusätzlich zum Speicher einen Programmleser auf. Die Übernahme des Steuerprogrammes erfolgt dann in der Weise, daß Lochkarte, Lochstreifen oder Magnetkarte in die Steuervorrichtung eingegeben und das gespeicherte Programm auf den internen Speicher der Steuervorrichtung übernommen wird. Es ist auch möglich, den Speicher der Steuervorrichtung austauschbar zu gestalten, so daß dieser Speicher direkt auf dem externen Programmiergerät vorprogrammiert werden kann. Eine separate Leseeinrichtung entfällt dann; die Programmübernahme wird auf einen Austausch des Speichers reduziert.

Weitere Einzelheiten der Erfindung werden in den nachfolgenden Figurenbeschreibungen von Ausführungsbeispielen anhand der Zeichnung erläutert. Es zeigt

Fig. 1 ein Prinzipschaltbild einer Vorrichtung nach der Erfindung,

Fig. 2 ein Blockdiagramm eines speziellen Programmiergerätes mit Kreuzschienenverteiler,

Fig. 3 ein Blockschaltbild eines speziellen Steuergerätes, bei dem zusätzlich Pumpenantriebssteuerung und -berwachung der Mikrodosiereinheit integriert sind und

Fig. 4 eine perspektivische Außenansicht einer Vorrichtung nach Fig. 1 mit Programmiergerät, Registriergerät sowie Steuervorrichtung und Mikrodosiereinheit, die aber abweichend von Fig. 1 durch eine Leitungsverbindung verbunden sind.

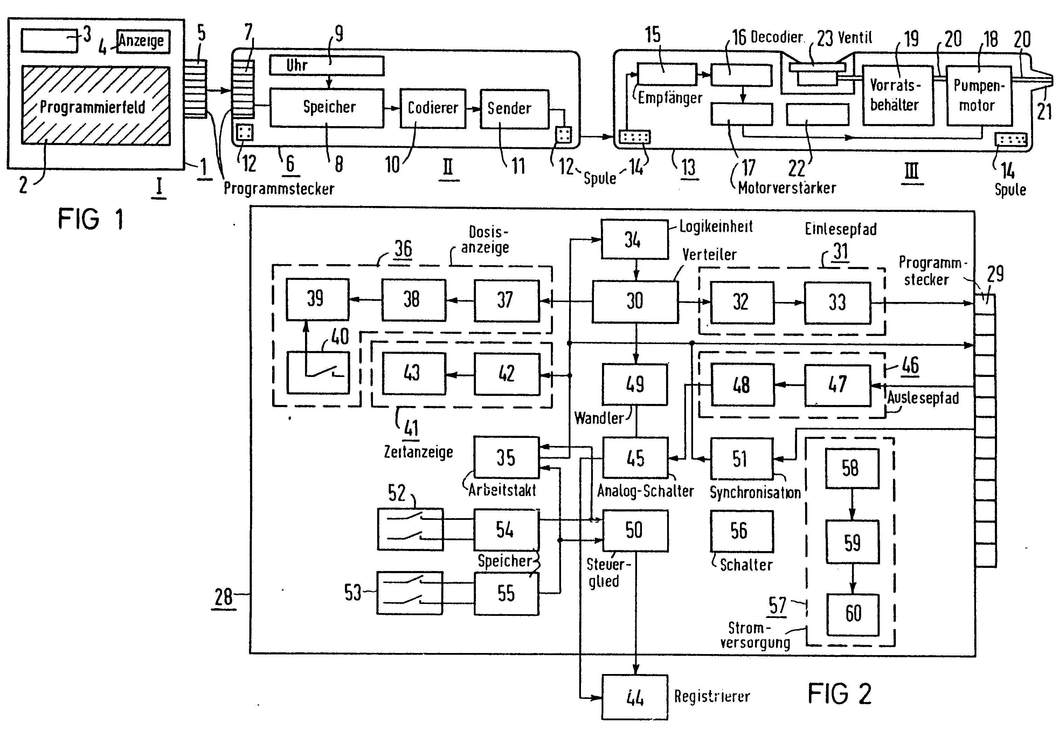

In der Fig. 1 ist mit I ein externes Programmiergerät, mit II eine Steuervorrichtung und mit III die Mikrodosiereinheit bezeichnet. Die Einzelgeräte I und II werden lediglich kurzzeitig zwecks Übernahme des Programmes mechanisch und elektrisch verbunden, während die Einzelgeräte II und III bei Funktion der Mikrodosiereinheit zur fortwährenden Signalübertragung verbunden sind. Dabei erfolgt die Signalübertragung über eine direkte Leitung oder drahtlos mittels Fernsteuerung. Insbesondere wird im Ausführungsbeispiel nach Fig. 1 eine induktive Signalübertragung angewendet. Das Gerät I beinhaltet ein Gehäuse 1 mit einem elektrischen Verteiler, insbesondere Kreuzschienenverteiler. Das Bezugszeichen 2 kennzeichnet dabei das Programmierfeld des Signalverteilers. Weiter sind Sicht- und Kontrollanzeigeeinheiten 3 und 4 und ein Programmierstecker 5 am Gehäuse 1 angeordnet. Das Gerät II besteht aus dem Gehäuse 6, das äußerlich eine zum Stecker 5 passende Programmiersteckdose 7 aufweist. Das Gehäuse 6 umfaßt als wesentliches Bauteil einen Speicher 8 zur Speicherung des mittels Programmiergerät I vorprogrammierten Tagesprogramms. Dem Programmspeicher 8 sind eine Quarzuhr 9 als Zeitgeber zugeordnet sowie ein Signalcodierer 10 mit nachgeschaltetem Sender 11 und Sendespule 12 nachgeschaltet, durch die das vom Programmierspeicher abgerufene Signal für die Übertragung aufbereitet wird. Die Sendespule 12 verläuft über die gesamte Grundfläche des Gehäuses 6 und ist an den gegenüberliegenden Ecken im Schnitt angedeutet.

Das Gehäuse der Mikrodosiereinheit III ist mit 13 bezeichnet. Es enthält in Analogie zur Steuervorrichtung I eine entsprechende Empfängerspule 14 auf der Grundfläche mit nächgeschaltetem elektronischem Empfänger 15 und Decodierer 16. Über den Decodierer 16 wird ein Motorverstärker 17 angesteuert, mit dem der Antriebsmotor einer mechanischen Pumpe 18 betrieben wird. Mittels Pumpe 18 wird aus einem Vorratsbehälter 19 das flüssige Insulin über eine Verbindungsleitung 20 zu einem Katheteranschluß 21 am Gerätegehäuse 13 befördert und in den Patientenkörper abgegeben. Das Gehäuse 13 der Mikrodosiereinheit III enthält weiterhin noch eine Batterie 22 als Energiequelle für den Pumpenantrieb sowie ein Nachfüllventil 23, über das mittels Spritze durch eine sich selbsttätig schließende Membran — bei implantierter Mikrodosiereinheit gegebenenfalls transkutan — Insulin in den Vorratsbehälter nachgefüllt werden kann.

In der Fig. 2 umfaßt das Programmiergerät mit einem Gehäuse 28 und einem Programmstecker 29 die elektrischen bzw. elektronischen Bauteile 30 bis 60 in nachfolgend beschriebener Schaltung: Einem elektrischen Kreuzschienenverteiler 30 ist ein Lesepfad 31 zugeordnet, der aus einem Raten-Binär-Wandler 32 und nachgeschaltetem Pegelkonverter 33 besteht. Über eine Ansteuerlogik 34, die von einem Taktgenerator 35 zur Erzeugung eines Arbeits- und Schiebetaktes angesteuert wird, wird mittels Schieberegistern der auf dem Kreuzschienenverteiler 30 manuell eingestellte elektrische Signalwert abgenommen und mittels Wandler 32 in ein Binärsignal gewandelt. Mittels Pegelkonverter 33 wird das Binärsignal an das erforderliche Signalniveau angepaßt, welches dann vom Steuergerät II direkt übernommen werden kann. Die auf dem Kreuzschienenverteiler 30 manuell eingestellten elektrischen Signalwerte werden weiterhin auf eine Einheit 36 zur Dosisanzeige gegeben. Diese Einheit 36 besteht im einzelnen aus einer digitalen Addierschaltung 37 mit nachgeschaltetem Verteiler und Treiber 38 als Anpaßteil und Anzeigeeinheit 39. Auf der Anzeigeeinheit 39 wird also digital die aufsummierte vorprogrammierte Tagesmenge in Insulineinheiten angezeigt. Mittels Taste 40 kann die Anzeige auf Null zurückgestellt werden. Vom Generator 35 wird weiter ein Takt für eine Einheit 41 zur Zeitangabe abgenommen, die aus einem Treiber 42 als Anpaßteil mit nachgeschalteter digitaler Anzeige 43 besteht.

Das auf dem Kreuzschienenverteiler manuell vorprogrammierte bzw. im Speicher der Steuervorrichtung gespeicherte Tagesprofil der Dosierung kann bei Bedarf als Analogsignal auf einem externen Registriergerät, das mit dem Bezugszeichen 44 angedeutet ist, aufgezeichnet werden. Dazu wird über einen Analogschalter 45 entweder ein Auslesepfad 46, der an den Speicher des Steuergerätes angeschlossen werden kann und im einzelnen aus einem Pegelkonverter 47 mit nachgeschaltetem Raten-Spannungs-Wandler 48 besteht, oder ein direkt an den Kreuzschienenverteiler 30 angeschalteter Raten-Spannungs-Wandler 49 auf das Meßwerk des Registriergerätes 44 gegeben. Der Zeitvorschub des Registriergerätes 44 wird dabei synchron von der Einheit 50 gesteuert. Zur Synchronisierung des mittels Kreuzschienenverteiler 30 vorgegebenen und gegebenenfalls im Speicher des Steuergerätes gespeicherten Programms dient eine Synchronisationseinheit 51. Weiterhin weist das Programmiergerät nach Fig. 2 Betätigungsschalter 52 und 53 auf, die über Befehlsspeicher 54 und 55 auf den Generator 35 zur Erzeugung des Schiebetaktes bzw. auf die Einheit 50 zur Steuerung des Registriergerätes einwirken. Mittels der Schalter 52 und 54 kann die beschriebene Logikschaltung derart gesteuert werden, daß wahlweise ganze Tagesprofile oder Einzelzeitschritte in den Speicher des Steuergerätes II ein- bzw. ausgelesen werden. Außerdem wird über Schalter 53 die Quarzuhr des Steuergerätes II auf die aktuelle Tageszeit eingestellt. Der Schalter 53 ist als Tastschalter ausgebildet, wobei mit Betätigung die Anzeige 41 und Quarzuhr des Steuergerätes II entsprechend dem Zeitraster des Kreuzschienenverteilers 30 um jeweils 30 Minuten weitergetastet wird. Mit 56 ist ein Schalter zum Einschalten der Spannungsversorgung des Gerätes für oben beschriebene Funktionen bezeichnet. Hierzu ist dem Gerät noch eine Einheit zur Stromversorgung 57 zugeordnet, die aus einem Akkumulator 58 mit Spannungsüberwachung 59 und Anzeige sowie einer Einheit zur Spannungsstabilisierung 60 besteht.

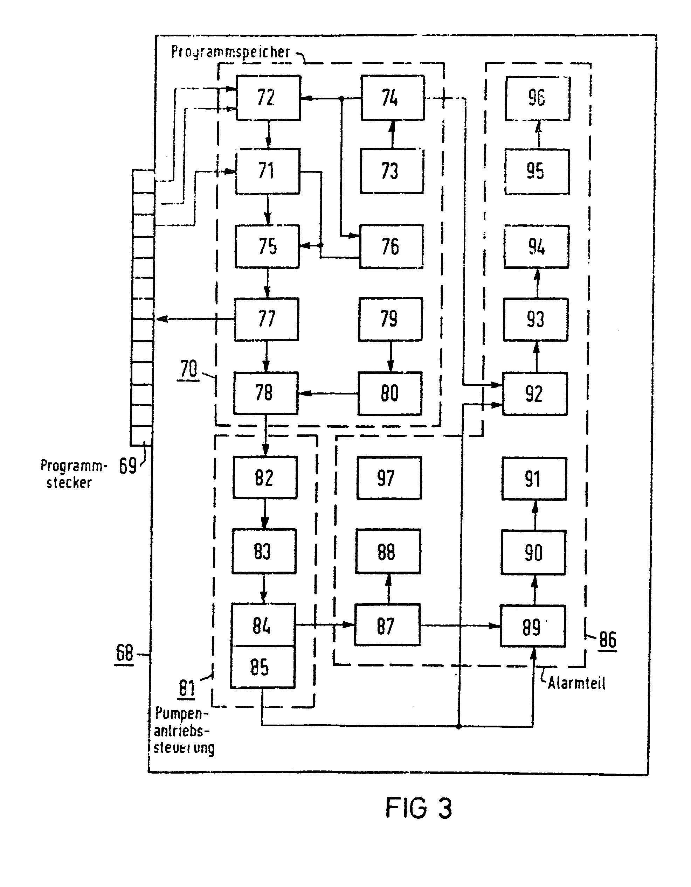

In der Fig. 3 umfaßt das Steuergerät mit im Gehäuse 68 integrierter Pumpenantriebssteuerung und Überwachung neben dem Programmstecker 69 die elektrischen bzw. elektronischen Bauteile 70 bis 97: Ein Programmspeicher 70 wird durch einen digitalen Halbleiterspeicher 71, ein sog. RAM, gebildet, dem eine Logikschaltung zur Adressierung und Auslesen der Werte zugeordnet ist. Dem Speicher 71 ist ein Adreßzähler 72 vorgeschaltet, der beim Einlesen eines Programms vom Einlesepfad des Programmiergerätes nach Fig. 2 angesteuert wird. Der Adreßzähler 72 wird gleichzeitig von einer Quarzuhr 73 über einen Frequenzteiler 74 angesteuert. Dem Speicher 71 ist ein Zwischenspeicher 75 für 4 bit nachgeschaltet. Da am Programmiergerät Zeitschritte von 30 Minuten vorprogrammiert werden können, wird jeweils nach 30 Minuten vom eigentlichen Programmspeicher 71 der anstehende Wert auf den Zwischenspeicher 75 gegeben. Zur Schaltsteuerung dient ein Monoflop 76, das vom Frequenzteiler 74 angesteuert wird und mittels Schaltsignal den anstehenden Wert vom Speicher 71 auf den Zwischenspeicher 75 überträgt. Diese Maßnahme hat den Vorteil, daß das RAM 71 nicht ständig an die Stromversorgung angeschaltet sein muß. Dem Zwischenspeicher ist ein BCD-Decodierer 77 nachgeschaltet, der das Steuersignal als Dezimalwert zurückwandelt. Über eine Torschaltung 78 wird gleichzeitig der Takt eines Taktgebers 79 mit nachfolgendem Binärteiler 80 auf die Pumpenantriebssteuerung gegeben.

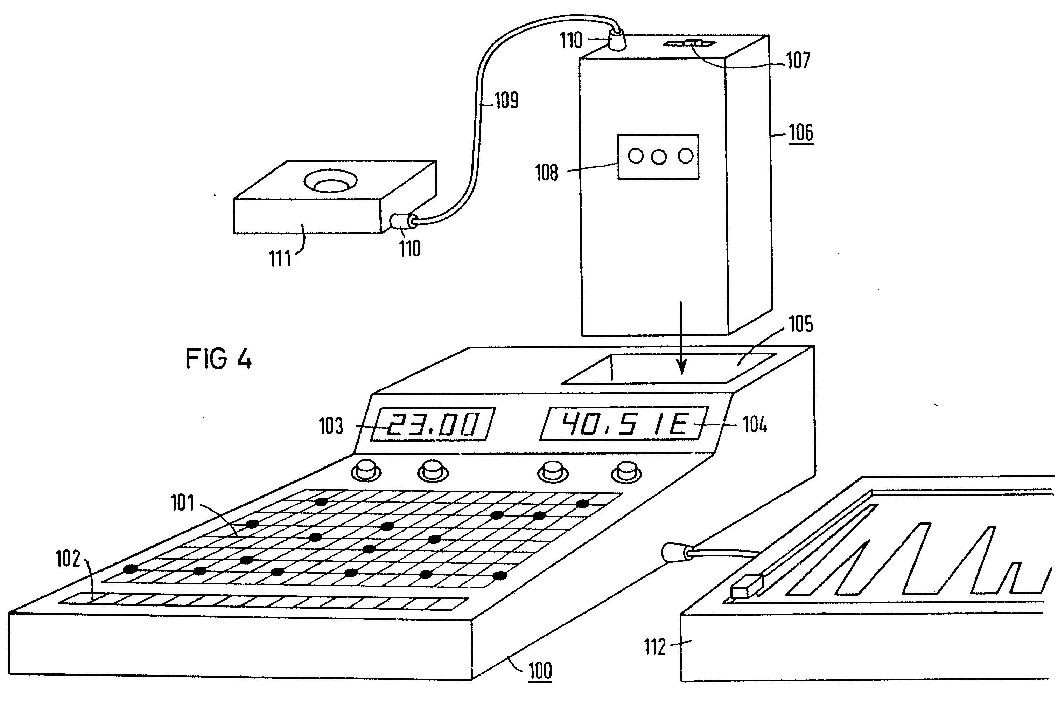

Im speziellen Ausführungsbeispiel nach Fig. 4 ist die Pumpenantriebssteuerung 81 für die Mikrodosiereinheit im Steuergerät integriert. Das Signal der Torschaltung 78 setzt ein Monoflop 82 zur Erzeugung eines Motorpulses. Über eine Treiberstufe 83 wird ein Schrittmotor 84 als Antrieb einer Pumpe, die beispielsweise als Rollenpumpe in der deutschen Patentanmeldung P 2 651 962 beschrieben ist, angesteuert. Mittels Schrittmotor 84 wird dabei im einzelnen der Pumpenantrieb für die Rollenpumpe in Gang gesetzt, wobei der Lauf der Pumpenrollen durch Schließen eines Reed-Kontaktes 85 überwacht wird. Mittels Alarmteil 86 kann nun die Funktion der Mikrodosiereinheit III überwacht werden. Der Monoflop 87 erzeugt beim Lauf des Schrittmotors 84 entsprechend der Motorfrequenz f Rechteckimpulse, die auf einer Anzeigeeinheit 88 als Impulse angezeigt werden. Die Rechteckimpulse werden gleichzeitig auf eine weitere Teiler- bzw. Zählerstufe 89 gegeben und gezählt. Da die Anzahl der durch den Reed-Kontakt 85 erzeugten Impulse bei korrektem Lauf der Rollenpumpe mit der Zahl der Schrittmotorimpulse korreliert ist, kann durch Vergleich der vom Steuergerät erzeugten Schrittmotorimpulse mit den Impulsen des Reed-Kontaktes 85 die Pumpfunktion überprüft werden. Nach Decodierung in der Erkennungseinheit 90 wird gegebenenfalls an der Anzeigeeinheit 91 ein mechanischer Defekt der Rollenpumpe — bei dem der Pumpenantrieb zwar Schrittmotorimpulse erhält, aber keine Rollenbewegung erfolgt — angezeigt. Die vom Reed-Kontakt 85 erzeugten Signalimpulse werden weiterhin auf eine Teiler- und Zählerstufe 92 gegeben, die vom Frequenzteiler 74 der Quarzuhr 73 jeweils bei Tagesbeginn automatisch auf Null gesetzt wird. Die Zahl der Reed-Impulse ist ein direktes Maß für die Menge der geförderten Flüssigkeit; es kann so — bei entsprechender Eichung in Insulineinheiten — die Zahl der von der Mikrodosiereinheit tatsächlich geförderten Insulineinheiten überprüft werden. Da mittels Mikrodosiereinheit III eine vorgegebene Gesamtinfusionsmenge für einen Patienten an Insulineinheiten pro Tag nicht überschritten werden soll, wird nach Decodierung des Signals mittels Einheit 93 auf der Anzeigeeinheit 94 gegebenenfalls ein Alarm angezeigt. Der Alarmteil 86 weist zusätzlich eine Anzeigeeinheit 96 für den Spannungszustand der Batterie 95 der Mikrodosiereinheit auf. Den Anzeigeeinheiten 91, 94 und 96 ist ein akustischer Alarmgeber 97 zur Erzeugung eines Pieptones zugeordnet.

In der Fig. 4 ist das externe Programmiergerät mit 100 bezeichnet. Es ist in etwa in Form einer Schreibmaschine ausgebildet, wobei ein Programmierfeld 101 mit 48 Spalten und 7 Zeilen die Bedienungsebene einnimmt. Mit einem derartigen Programmierfeld 101 können also 7 diskrete Infusionsraten mit einem Zeitraster von 30 Minuten eingestellt werden. Unterhalb des Programmierfeldes 101 ist eine Signalleiste 102 den 48 Spalten des Programmierfeldes 101 zugeordnet, auf der der Arzt Signale vorprogrammieren kann, die den Patienten als akustisches Signal zur vorbestimmten Tageszeit die Einnahme von Mahlzeiten u. dgl. signalisieren. Auf dem pultförmigen Teil sind Digitalanzeigeeinheiten 103, 104 zur Anzeige der Tageszeit und der Gesamttagesdosierung sowie gegebenenfalls weitere Kontrolleinheiten und Bedienelemente angeordnet. Die Horizontalfläche des Pultteiles weist einen Einführschacht 105 für die Steuervorrichtung im Gerätegehäuse 106 auf, in den bei Programmübernahme bzw. Auslesen des Programmes das Steuergerät 106 manuell eingeschoben und mittels Programmierstecker elektrisch verbunden wird. Nach Einschieben des Steuergerätes 106 erfolgt dabei eine automatische Zeitsynchronisation zwischen Steuergerät 106 und Programmiergerät 100.

Das Steuergerät 106 weist in weiterer zweckmäßiger Ausgestaltung ein Bedienelement 107 auf, mit dem über in Potentiometer eine Eichung der mittels Programmiergerät 100 vorprogrammierten, relativen Amplituden der Abgaberate in absolute Insulineinheiten (IE) pro Stunde erfolgen kann. Mit diesem Bedienelement 107 kann der Patient im Notfall das Nieveau der Tadesdosierung selbständig verändern und die vorprogrammierte Abgaberate an eine veränderte Situation anpassen. Am Steuergerät 106 ist weiterhin ein Anzeigefeld 108 zur optischen Alarmanzeige bei Fehlfunktionen aufgeordnet. Über ein elektrisches Kabel 109 ist das Steuergerät 106 mit der Mikrodosiereinheit 111 verbunden. Mikrodosiereinheit 111 und Steuergerät 106 haben jeweils lösbare Kabelanschlüsse 110, so daß während der Programmübernahme Steuergerät 106 und Mikrodosiereinheit 111 nicht verbunden sind. Dem Programmiergerät 100 ist ein Registriergerät 112 zur Analoganzeige des am Kreuzschienenverteiler 101 des Programmiergerätes 100 manuell eingesteckten Steuerprogramms bzw. zum Auslesen des im Steuergerät 106 gespeicherten Programms zugeordnet. Als Registriergerät 112 kann ein gewöhnlicher Zeitschreiber verwendet werden.

In den beschriebenen Ausführungsbeispielen wird das Tagesprofil der Dosierung vom Arzt jeweils am separaten Programmiergerät manuell einprogrammiert und durch kurzzeitige Verbindung von Steuergerät 106 mit dem Programmiergerät 100 das Programm in den Speicher des Steuergerätes 106 übernommen. In weiteren Ausbildungen der Erfindung kann nun die Programmierung auch derart erfolgen, daß am Programmiergerät bei der Programmierung die Information direkt auf einen Programmträger gebracht wird. Es können so beispielsweise Lochkarten, Lochstreifen oder Magnetkarten codiert werden, die von einer zugehörigen Leseeinheit im Steuergerät 106 gelesen und so in den internen Speicher übernommen werden. Derartige Programmträger eignen sich insbesondere für die Dokumentierung und den Aufbau einer Programmbibliothek. Speziell bei Lochstreifen und Lochkarten kann nun die Codierung auch derart vorgenommen werden, daß die Lochstanzung eine »quasi-analoge« Darstellung des Tagesprofils wiedergibt. Dadurch können solche Programmträger vom Benutzer schon optisch klassifiziert werden.

Da inzwischen auch programmierbare Halbleiterspeicher in Chipform bekannt sind, ist auch eine weitere Abwandlung der erfindungsgemäßen Vorrichtung möglich, bei der die Speichereinheit im Steuergerät 106 austauschbar ist. Ein Speicher-Chip dient dann sowohl als Programmierträger im Programmiergerät als auch als internes Speichermittel in der Steuervorrichtung. In einem solchen Fall wird aus einzelnen Speicher-Chips eine Bibliothek aufgebaut, aus der — gegebenenfalls auch ohne weitere Nachprogrammierung — ein Speicher-Chip mit einem für den Patienten geeigneten Programm entnommen und im Steuergerät eingesetzt werden kann. Es entfällt also eine spezielle Übernahme des Programms in den internen Speicher des Steuergerätes 106 durch Lesen mit Ausleseeinheiten. Auch derartige Abwandlungen fallen mit unter die Erfindung.

**Patentansprüche**

1. Vorrichtung zur vorprogrammierbaren Infusion von Flüssigkeiten in einen Patientenkörper, insbesondere zur Insulinverabreichung bei der Diabetes-Therapie, bestehent aus einer Mikrodosiereinheit für die Flüssigkeit sowie einer der Mikrodosiereinheit vorgeschalteten, programmierbaren und mit einem Programmspeicher für die Mikrodosiereinheit versehenen Steuervorrichtung, in die ein Steuerprogramm für den Verlauf der Infusionsrate zwischen vorgegebenen Zeitpunkten eines 24-Stunden-Rhythmus manuell einprogrammierbar ist, dadurch gekennzeichnet, daß zur Programmierung ein von der Steuervorrichtung (II; 6, 68, 106) getrenntes, eigenes Programmiergerät (I; 1, 28, 100) vorhanden ist, und daß der Speicher (8, 70) für das vorprogrammierte Steuerprogramm der Steuervorrichtung (II; 6, 68, 106) als Programmgeber für die Mikrodosiereinheit (III; 13, 111) vorgesehen und ausschließlich zur Übernahme des vorprogrammierten Steuerprogramms kurzzeitig mit dem externen Programmiergerät (I; 1, 28, 100) verbindbar ist.

2. Vorrichtung nach Anspruch 1, dadurch gekennzeichnet, daß die Verbindung zur Übernahme des vorprogrammierten Steuerprogramms durch rein elektrische oder elektromechanische Anschaltung des Speichers (8, 70) der Steuervorrichtung (II, 6, 68, 106) an den das Steuerprogramm gebenden Teil des Programmiergerätes (I, 1, 28, 100) erfolgt.

3. Vorrichtung nach Anspruch 1, dadurch gekennzeichnet, daß die Verbindung zur Übernahme des vorprogrammierten Steuerprogramms über das Steuerprogramm beinhaltende und vom Programmiergerät (I; 1, 28, 100) gelieferte Programmträger erfolgt.

4. Vorrichtung nach Anspruch 1, dadurch gekennzeichnet, daß das Programmiergerät (I, 1, 28, 100) einen elektrischen Verteiler, beispielsweise Kreuzschienenverteiler (101), aufweist, dessen Zeilen definierte Infusionsraten und dessen Spalten bestimmte Tageszeiten des 24-Stunden-Rhythmus zugeordnet sind.

5. Vorrichtung nach Anspruch 1, dadurch gekennzeichnet, daß das Programmiergerät (I, 1, 28, 100) einen Infusionsratenschalter aufweist, mit dem in zeitlicher Folge definierte Abgaberaten entsprechend dem zeitlichen Tagesbedarf an Flüssigkeit vorgewählt werden können.

6. Vorrichtung nach Anspruch 3, dadurch gekennzeichnet, daß die Programmträger Lochkarten oder Lochstreifen sind, auf denen mittels geeigneter Codierung das Programm optisch erkennbar in quasi-analoger Darstellung gespeichert ist.

7. Vorrichtung nach Anspruch 3, dadurch gekennzeichnet, daß die Programmträger Magnetkarten sind.

8. Vorrichtung nach Anspruch 1 und 3, dadurch gekennzeichnet, daß mittels des Programmiergerätes (I, 1, 28, 100) ein Tagesprofil der Dosierung auf einen austauschbaren Speicher-Chip gebbar ist, der direkt in das Gerätegehäuse (6) der Steuervorrichtung (II, 6, 68, 106) als interner Speicher (8, 70) einsetzbar ist.

9. Vorrichtung nach Anspruch 1, dadurch gekennzeichnet, daß das mittels des Programmiergerätes (I, 1, 28, 100) vorprogrammierte und/oder das in der Steuervorrichtung (II, 6, 68, 106) gespeicherte Programm der Tagesdosierung als Analogsignal auf einem dem Programmiergerät (I, 1, 28, 100) zugeordneten Registriergerät (44, 112) darstellbar ist.

10. Vorrichtung nach Anspruch 1, dadurch gekennzeichnet, daß der Speicher (8, 70) ein digitaler elektronischer Speicher (71, 75) ist.

11. Vorrichtung nach einem der vorhergehenden Ansprüche, dadurch gekennzeichnet, daß das Programmiergerät (I, 1, 28, 100) Einheiten (36 bis 39, 104) zur Aufsummierung und Anzeige der Gesamttagesdosis, insbesondere direkt in Insulineinheiten (IE) bei der Insulinverabreichung, entsprechend dem vorprogrammierten Tagesprofil aufweist.

12. Vorrichtung nach Anspruch 1, dadurch gekennzeichnet, daß das Programmiergerät (I, 100) eine Einheit (102) zur zeitlichen Vorprogrammierung von akustischen Signalen aufweist, die an der Steuervorrichtung (II, 6, 68, 106) dem Patienten beispielsweise die Einnahme von Mahlzeiten signalisieren.

13. Vorrichtung nach Anspruch 1, dadurch gekennzeichnet, daß die Steuervorrichtung (II, 6, 68, 106) eine quarzgesteuerte Uhr (73) als Zeitgeber aufweist.

14. Vorrichtung nach Anspruch 1, dadurch gekennzeichnet, daß dem Programmiergerät (I, 1, 28, 100) und/oder der Steuervorrichtung (II, 6, 68, 106) eine Einheit (51) zur Anpassung des am Programmiergerät (I, 1, 28, 100) einprogrammierten Tagesprofils der Dosierung an die aktuelle Tageszeit zugeordnet ist.

15. Vorrichtung nach Anspruch 1, dadurch gekennzeichnet, daß die Steuervorrichtung (II, 6, 68, 106) ein Betätigungsglied (107) mit einem Potentiometer zur Eichung und gegebenenfalls Abänderung der mittels des Programmiergerätes (I, 1, 28, 100) vorgegebenen relativen Abgaberate einer Flüssigkeit in eine absolute Infusionsrate, beispielsweise Insulineinheiten pro Stunde (IE/h), aufweist.

16. Vorrichtung nach einem der Ansprüche 13 bis 15, dadurch gekennzeichnet, daß der Steuervorrichtung (I) eine Einheit (86) zur Überwachung der Mikrodosiereinheit (III) und zur Abgabe eines optischen und/oder akustischen Alarmsignals zugeordnet ist.

17. Vorrichtung nach Anspruch 1, dadurch gekennzeichnet, daß die Steuervorrichtung (II, 6, 68, 106) in einem von der Mikrodosiereinheit (III, 13, 111) getrennten Gerätegehäuse (6) untergebracht ist und daß die Steuervorrichtung (II, 68, 106) und die Mikrodosiereinheit (III, 13, 111) zur Übertragung von Steuersignalen verbindbar sind.

18. Vorrichtung nach Anspruch 17, dadurch gekennzeichnet, daß die Steuervorrichtung (II, 106) und Mikrodosiereinheit (III, 111) durch eine Leitung (109) zur Übertragung der Steuersignale verbunden sind.

19. Vorrichtung nach Anspruch 17, dadurch gekennzeichnet, daß die Steuersignale von der Steuervorrichtung (II, 6, 68) zur Mikrodosiereinheit (III, 111) drahtlos mittels Fernsteuerung, vorzugsweise induktiv, übertragen werden.

## Claims

1. Device for the pre-programmable infusion of liquids into a patient's body, in particular für feeding insulin in diabetes therapy, composed of a micro-dose unit for the liquid and of a programmable control device which precedes the micro-dose unit, is provided with a programme store for the micro-dose unit, and into which it is possible to manually programme a control programme for the course of the infusion rate between predetermined times of a 24-hour cycle, characterised in that for programming, a special programming device (I; 1, 28, 100) is provided which is isolated from the control device (II; 6, 68, 100), and that the store (8, 70) for the pre-programmed control programme of the control device (II; 6, 68, 106) serves as programmer for the micro-dose unit (III; 13, 111) and, exclusively for the transfer of the pre-programmed control programme, may be temporarily connected to the external programming device (I; 1, 28, 100).

2. Device as claimed in Claim 1, characterised in that the connection for the transfer of the pre-programmed control programme takes place by means of purely electrical or electromechanical connection of the store (8, 70) of the control device (II; 6, 68, 106) to that part of the programming device (I; 1, 28, 100) which supplies the control programme.

3. Device as claimed in Claim 1, characterised in that the connection for the transfer of the pre-programmed control programme takes place via programme carries which contain the control programme and which are supplied by the programming device (I; 1, 28, 100).

4. Device as claimed in Claim 1, characterised in that the programming device (I; 1, 28, 100) possesses an electrical distributor, for example a cross-bar distributor (101), the rows of which are assigned to determinate infusion rates and the columns of which are assigned to specific times of day within the 24-hour cycle.

5. Device as claimed in Claim 1, characterised in that the programming device (I; 1, 28, 100) possesses an infusion rate switch by means of which it ist possible to preselect determinate output rates, in a time sequence in accordance with the daily requirement of liquid.

6. Device as claimed in Claim 3, characterised in that the programme carriers consist of punched cards or punched strips on which the programme is stored by means of suitable coding so as to be optically detectable in a quasi-analogue representation.

7. Device as claimed in Claim 3, characterised in that the programme carriers consist of magnetic cards.

8. Device as claimed in Claim 1 and 3, characterised in that by means of the programming device (I; 1, 28, 100) it is possible to produce a daily profile of the dose on an interchangeable storage chip which can be inserted directly into the device housing (6) of the control device (II; 6, 68, 106) as an internal store (8, 70).

9. Device as claimed in Claim 1, characterised in that the programme of the daily dose which has been pre-programmed by means of the programming device (I; 1, 28, 100) and/or which has been stored in the control device (II, 6, 68, 106) can be represented as an analogue signal on a recording device (44, 112) assigned to the programming device (I; 1, 28, 100).

10. Device as claimed in Claim 1, characterised in that the store (8, 70) consists of a digital electronic store (71, 85).

11. Device as claimed in one of the preceding Claims, characterised in that the programming device (I; 1, 28, 100) comprises units (36 to 39, 104) for adding up and displaying the total daily dose, in particular directly in insulin units (IE) in the case of insulin feeding, in accordance with the pre-programming daily profile.

12. Device as claimed in Claim 1, characterised in that the programming device, (I; 100) possesses a unit (102) for the timed pre-programming of acoustic signals which signal, for example the taking of mealtimes, to the patient on the control device (II; 6, 68, 106).

13. Device as claimed in Claim 1, characterised in that the control device (II; 6, 68, 106) possesses a quartz controlled clock (73) as timer.

14. Device as claimed in Claim 1, characterised in that the programming device (I; 1, 28, 100) and/or the control device (II; 6, 68, 106) is assigned a unit (51) which serves to match the daily dosage profile which has been programmed on the programming device (I; 1, 28, 100) to the current time of day.

15. Device as claimed in Claim 1, characterised in that the control device (II; 6, 68, 106) possesses an actuating element (107) comprising a potentiometer for calibration and possibly modification of the relative emission rate of a liquid, which has been predetermined by means of the programming device (I; 1, 28, 100), into an absolute infusion rate, for example insulin units per hour (IE/h).

16. Device as claimed in one of the Claims 13 to 15, characterised in that the control device (I) is assigned a unit (86) which serves to monitor the micro-dose unit (III) and to emit an optical and/or acoustic alarm signal.

17. Device as claimed in Claim 1, characterised in that the control device (II; 6, 68, 106) is accommodated in a device housing (6) which is separate form the micro-dose unit (III; 13, 111), and that the control device (II, 6, 68, 106) and the micro-dose unit (III; 13, 111) can be connected for the transmission of control signals.

18. Device as claimed in Claim 17, characterised in that the control device (II; 106) and the micro-dose unit (III; 111) are connected by means of a line (109) for the transmission of the control signals.

19. Device as claimed in Claim 17, characterised in that the control signals are transmitted from the control device (II; 6, 68) to the micro-dose unit (III; 111) in wireless fashion by means of remote control, and preferably inductively.

**Revendications**

1. Dispositif pour infuser de façon programmable à l'avance des liquides dans le corps d'un patient, plus particulièrement pour administrer de l'insuline dans le traitement du diabète, constitué par une unité de microdosage pour le liquide et par une unité de commande qui est montée en amont de l'unité de microdosage, qui est programmable à l'avance et qui est pourvue d'une mémoire de programme pour l'unité de microdosage et dans laquelle peut être programmé manuellement un programme de commande pour l'allure des quantités à infuser entre deux instants prédéterminés d'un rythme de 24 heures, caractérisé par le fait qu'il est prévu, pour la programmation, un appareil propre de programmation (I; 1, 28, 100) et distinct du dispositif de commande (II; 6, 68, 106), et que la mémoire (8, 70) pour le programme de commande préprogrammé du dispositif de commande (II; 6, 68, 106) est prévue comme générateur de programme pour l'unité de microdosage (III; 13, 111) et est susceptible d'être reliée pour une courte durée à l'appareil extérieur de programmation (I; 1, 28, 100) pour prendre en charge le programme de commande préprogrammé.

2. Dispositif selon la revendication 1, caractérisé par le fait que la liaison ou la prise en charge des programmes de commande préprogrammés est opérée par le branchement par voie purement électrique ou électromécanique de la mémoire (8, 70) du dispositif de commande (II; 6, 68, 106) à la partie de l'appareil de programmation (I; 1, 28, 100) qui fournit le programme de commande.

3. Dispositif selon la revendication 1, caractérisé par le fait que le branchement ou la prise en charge du programme de commande préprogrammé est opérée par l'intermédiaire du support de programme contenant le programme de commande et fourni par l'appareil de pgrogrammation (I; 1, 28, 100).

4. Dispositif selon la revendication 1, caractérisé par le fait que l'appareil de programmation (I; 1, 28, 100) comporte un répartiteur électrique, par exemple un répartieur à barres croisée (101) aux lignes duquel sont associées des quantités définies à administrer et aux colonnes duquel sont associés temps déterminés d'un rythme de 24 heures.

5. Dispositif selon la revendication 1, caractérisé par le fait que l'appareil de programmation (I; 1, 28, 100) comporte un commutateur de quantités à infuser, avec lequel on peut choisir à l'avance les doses de liquide à aministrer successivement dans le temps, en fonction du besoin quotidien.

6. Dispositif selon la revendication 3, caractérisé par le fait que les supports de programme sont des cartes perforées ou des bandes perforées sur lesquelles est mémorisé, à l'aide d'un codage approprié, et sous une forme quasi-analogique, le programme qui est susceptible d'être lu par voie optique.

7. Dispositif selon la revendication 3, caractérisé par le fait ques les supports de programme sont des cartes magnétiques.

8. Dispositif selon la revendication 1 et 3, caractérisé par le fait qu'à l'aide du support de programmation (I; 1, 28, 100), le profil d'une journée peut être programmé sur une puce de mémoire remplaçable et susceptible d'être montée directement dans le boitier (6) du dispositif de commande (II; 6, 68, 106) en tant que mémoire interne (8, 70).

9. Dispositif selon la revendication 1, caractérisé par le fait que le programme de dosage quotidien, préprogrammé à l'aide de l'appareil de programmation (I; 1, 28, 100) et/ou mémorisé dans le dispositif de commande (II; 6, 68, 106), est susceptible d'être représenté comme un signal analogique sur un appareil enregistreur (44, 112) associé à l'appareil de programmation (I; 1, 28, 100).

10. Dispositif selon la revendication 1, caractérisé par le fait que la mémcire (8, 70) est une mémoire électronique (71, 75) du type numérique.

11. Dispositif selon l'une des revendications précédentes, caractérisé par le fait que l'appareil de programmation (I; 1, 28, 100) comporte des unités (36 à 39, 104) de sommation et d'affichage de la dose totale, de préférence directement en unités d'insuline (IE), lors de l'administration de l'inusline, selon le profil quotidien préprogrammé.

12. Dispositif selon la revendication 1, caractérisé par le fait que l'appareil de programmation (I; 100) comporte une unité (102) pour préprogrammer, en fonction du temps, des signaux acoustiques qui signalent au patient, au niveau du dispositif de commande (II; 6, 68, 106), par exemple l'absorption de repas.

13. Dispositif selon la revendication 1, caractérisé par le fait que le dispositif de contrôle (II; 6, 68, 106) comporte une horloge à quartz (73) servant de générateur de signaux de temps.

14. Dispositif selon la revendication 1, caractérisé par le fait qu'au dispositif de programmation (I; 1, 28, 100) et/ou au dispositif de commande (II; 6, 68, 106) est associée une unité (51) pour adapter le profil quotidien de dosage programmé dans l'appareil de programmation (I; 1, 28, 100) au temps effectif de la journée.

15. Dispositif selon la revendication 1, caractérisé par le fait que le dispositif de commande (II; 6, 68, 106) comporte un organe de commande (107) à potentiomètre pour étalonner et éventuellement modifier la quantité de liquide relative déterminée à l'avance par l'appareil de programmation (I; 1, 28, 100) en une quantité absolue à infuser par exemple d'unités d'insuline par heure (IE/h).

16. Dispositif selon l'une des revendications 13 à 15, caractérisé par le fait qu'au dispositif de commande (I) est associée une unité (83) servant à surveiller l'unité de microdosage (III) et à délivrer un signal d'alarme optique et/ou acoustique.

17. Dispositif selon la revendication 1, caractérisé par le fait que le dispositif de commande (II; 6, 68, 106) est logé dans un boitier (6) distinct de l'unité de microdosage (III; 13, 111), et que le dispositif de commande (II; 6, 68, 106) et l'unité de microdosage (III; 13, 111) sont susceptibles d'être liés pour transmettre des signaux de commande.

18. Dispositif selon la revendication 17, caractérisé par le fait que le dispositif de commande (II; 106) et l'unité de microdosage (III; 111) sont reliés entre eux par un conducteur (109) pour transmettre des signaux de commande.

19. Dispositif selon la revendication 17, caractérisé par le fait que les signaux de commande du dispositif de commande sont transmis à l'unité de microdosage (III; 111), par télécommande sans fil, de préférence inductive.

**FIG 1**

3  4 Anzeige  5  7  9 Uhr  15  16 Decodier.  23 Ventil  19  20  18  20

Programmierfeld

Speicher  Codierer  Sender

Empfänger

Vorrats-behälter  Pumpen-motor

2  1  12  6  8  10  II  11  12 Spule  14  13  17  22  III  14  21

Programmstecker  Motorverstärker  Spule

I

**FIG 2**

Dosis-anzeige  34 Logikeinheit  Einlesepfad

36  Verteiler  31  Programm-stecker  29

39  38  37  30  32  33

40  43  42  49  48  47  Auslesepfad

41  Wandler  46

Zeitanzeige  35  45  51  58

Arbeitstakt  Analog-Schalter  Synchronisation

52  59

54  50  56

53  55  Steuer-glied  Schalter  57  60

Speicher  Strom-versorgung

28  44 Registrierer

FIG 3

FIG 4